# EUROPEAN PATENT APPLICATION

(11) **EP 2 269 676 A2**
(43) Date of publication of application: **05.01.2011**
(21) Application number: 10012522.8
(22) Date of filing: 08.05.2001
(51) Int. Cl.: A61M 16/00, A61M 16/12

(54) **Apparatus for the administration of continuous positive airway pressure therapy**

(30) Priority: 12.05.2000 US 569898
(62) Divisional of application: 01933149.5
(71) Applicant: E.M.E. (Electro Medical Equipment) Ltd., Brighton, Sussex BN2 3QD (GB); Stenzler, Alex, Orange, CA 92869 (US)
(72) Inventor: Stenzler, Alex, Orange CA 92869 (US); Nilsson, Kjell, O., 831 45 Ostersund (SE)
(74) Representative: Richards, John

(57) **Abstract**

The apparatus centers on a driver (30) used in conjunction with a gas delivery. The driver (30) includes a valve (34), adapted to receive gas from a source, for regulating the flow of gas to the delivery device (10) in response to a control signal. A controller (36), connected to the valve (34), generates the control signal so that the valve (34) periodically modifies the flow of gas, thereby injecting a sigh cycle. A sensor (46) is connected to sense the airway pressure and generate a pressure signal representative of the airway pressure. The controller (36) periodically modifies the flow of gas in response to this pressure signal. It is especially preferred to initiate a sigh cycle when a drop in airway pressure is sensed.

## Description

### FIELD OF THE INVENTION

The present invention generally relates to methods and devices used to improve gas exchange during spontaneous breathing when the patient's ability to breath is impaired, and more particularly, to methods and equipment used for the administration of continuous positive airway pressure (CPAP) therapy.

### BACKGROUND OF THE INVENTION

For sick babies, the early hours of life are usually characterized by the need for respiratory or circulatory support. Premature babies are especially likely to have respiratory problems because their lungs have not had enough time to develop before birth. Such respiratory problems can include decreased pulmonary compliance, decreased functional residual capacity (FRC) and airway closure. Treatment of newborns requiring respiratory support is typically done in an intensive care environment.

Historically, in many countries, the initial treatment method prescribed for infants with respiratory problems is intubation and mechanical ventilation. Intubation involves placing a tube into the newborn's tracheal airway. Mechanical ventilation is a process which achieves a positive inflation of the lungs via the tube, thereby permitting gas to be exchanged in the lungs. For many years now, this treatment method has been followed by the use of a therapy called CPAP (continuous positive airway pressure) as a transition or weaning technique from mechanical ventilation to total independent breathing. Stated another way, once the respiratory problem, i.e., the disease or lung dysfunction, has resolved to the point where the baby can support its own ventilation, the baby is transitioned to a less invasive respiratory support. CPAP is a technique that applies a counterbalancing force of air pressure to the normal recoil of the lungs that would cause the alveoli to collapse. The purpose of the application of CPAP is to maintain a normal lung volume in an infant while allowing them to breathe on their own. It is also known to use CPAP as an initial treatment for babies with respiratory problems before resorting to intubation and mechanical ventilation.

CPAP utilizes slight positive pressure during the respiratory cycle in a spontaneously breathing baby to increase the volume of inspired air and to decrease the work and effort of breathing. This treatment can be applied by mouth, nose, or through ventilation tubes. Nasal CPAP, also referred to as NCPAP, is administered through nasal prongs (small cylinders placed into the infants nostrils) which are placed and secured in the infant's nose. Again, a small consistent positive pressure is used to increase the amount of air inhaled without increasing the work of breathing.

Since most newborns are preferential nose breathers, nasal prongs constitute a simple vehicle for the application of CPAP. Such nasal administered CPAP is referred to as NCPAP. One significant problem with early NCPAP equipment was the combination of high resistance to the breathing flow through the prongs and less effective pressure generators. This combination resulted in airway pressure instability which increased the work required for an infant to breathe, an undesirable condition. The unstable airway pressure also results in a less effective treatment in relation to the recruitment of alveoli and increasing lung capacity.

U.S. Patent Patent No. 5,193,532 - Nilsson, incorporated herein by reference, discloses a device for administering CPAP and in particular NCPAP. NCPAP systems generally include two basic elements, a "driver" and a "generator." The "driver" is a component that mixes dry medical air and oxygen and controls or meters its delivery to the infant patient. The driver also monitors the infant's airway pressure making it possible to evaluate whether the airway pressure remains at the level selected by the treating physician. The "generator" contains a miniature fluidic device, such as that disclosed in U.S. Patent No. 5,193,532, that performs two functions simultaneously. First, it controls and maintains a stable continuous positive airway pressure for the infant. Second, it minimizes the baby's exhalation effort.

In particular, during inspiration, the internal structure of the generator causes the flow of air or gas from the driver to accelerate or slightly elevate the pressure of incoming air, thereby reducing the work of breathing or effort required from the infant. During expiration, the internal structure of the generator causes the flow from the driver to "flip" or change direction and assist with the removal of air, again reducing the work of breathing. The change of direction of the pressurized flow of air or gas is referred to as the "fluidic flip." Due to the fluidic flip, this NCPAP equipment is able to maintain a stable positive pressure.

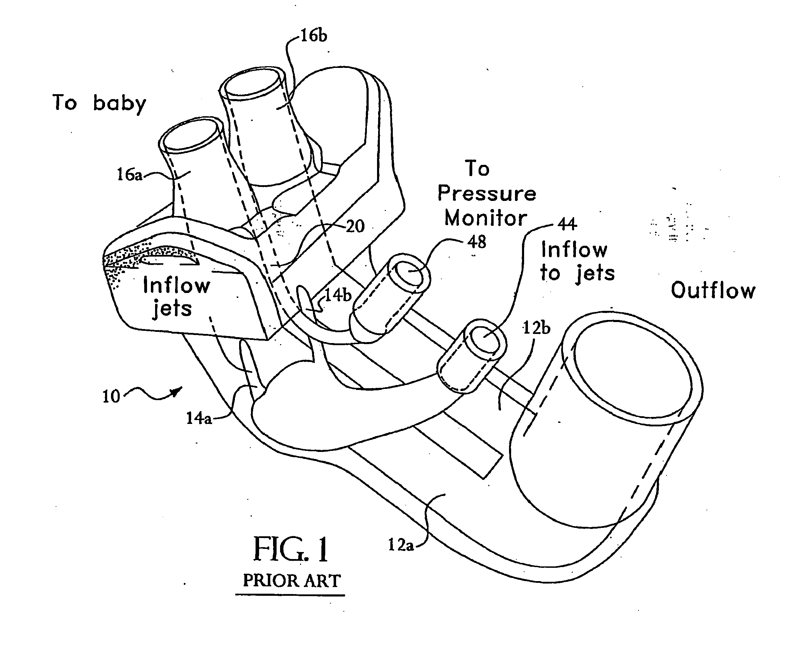

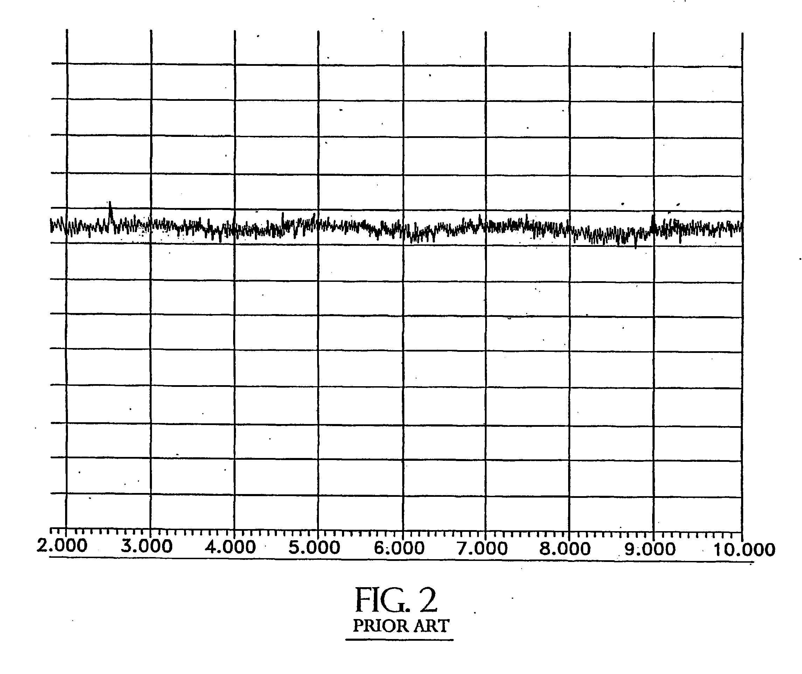

Presently, the generator which is the subject of U.S. Patent No. 5,193,532 is incorporated in a system manufactured by Electro Medical Equipment Ltd. of the United Kingdom, the assignee herein, and is called the INFANT FLOW™ System. An embodiment of this generator is generally depicted in Fig. 1 and an example of the ability of the INFANT FLOW™ System to maintain a stable positive airway pressure is depicted in Fig. 2. The INFANT FLOW™ System is believed to be in use in over 90% of UK hospitals and is seen as a cornerstone of treatment. Hospital in the United States are in the process of developing a similar level of confidence in neonatal NCPAP treatment.

Although NCPAP has been shown to provide significant benefits to newborns, there are benefits remaining to be realized. In particular, the present invention involves an improved NCPAP system that provides intermittent sighs or deep breaths during administration. It is believed that injecting sighs into administered CPAP will have several benefits, including, stimulating the respiratory center, stimulating the release of surfactant, and offloading respiratory work.

In the past, in connection with artificial respiration in adults, i.e., in relation to a device usually having a fixed respiratory pattern, it has been suggested to include sigh or deep breath cycles in the administration of such artificial respiration. In U.S. Patent No. 4,301,793 - Thompson, a portable respirator apparatus is disclosed. The respirator apparatus is shown to include a blower controlled by a control circuit mechanism. Periodically, for two or as many as six seconds each hour, the blower speed is increased, thereby increasing the peak pressure supplied to a patient. The respirator disclosed in U.S. Patent No. 4,301,793 operates in accordance with a fixed pattern to control breathing. Such a system is incapable of administering CPAP. By contrast, patients on CPAP control their own respiratory pattern which is varied.

CPAP has also been prescribed in the past for respiratory conditions such as sleep apnea and hypopnea. U.S. Patent No. 5,865,173 - Froehlich discloses a bi-level CPAP system in which pressure is regulated between a prescribed inspiratory positive airway pressure (IPAP) and a lower prescribed expiratory positive airway pressure.

Consequently, a need still exists for a system which is capable of injecting sighs during the administration of CPAP, and particularly during the administration of NCPAP.

### SUMMARY OF INVENTION

The above described problems are resolved and other advantages are achieved in novel methods and apparatus for administering continuous positive airway pressure (CPAP) therapy. In particular, a driver, adapted for use in connection with a gas delivery device for the generation of CPAP in a patient, is provided with controllable valve and a controller. The valve is connected to receive gas from a source and regulate the flow of the gas to the gas delivery device in response to a control signal. A controller generates the control signal so that the valve periodically modifies the flow of gas to the delivery device, thereby injecting a sigh cycle.

It is preferred to use a sensor to sense the pressure in the delivery device breathing channel and to generate a pressure signal representative of the pressure in the breathing channel. The controlled, connected to receive the pressure signal, generates the control signal in response to the pressure signal so that the gas flow is periodically modified. In such an embodiment, the controller is constructed to monitor the pressure signal, to determine when a drop is pressure is occurring and to cause the valve to modify the flow of gas in response to such pressure drop determination. Such a drop in pressure is a marker that inspiration is occurring.

However, it is not essential in the practice of the present invention to measure pressure in the breathing channel, i.e., airway pressure. It is only preferred that one determine the onset of inspiration. This condition could be determined many ways, for example, via a flow sensor measuring either flow rate or flow direction, an electrical impedance measuring device connected to measure chest wall expansion and contraction, diaphramatic EMG, inductance plethysmogaphy or any other volume or flow condition that would indicate inspiration.

It is also preferred for the controller to be a programmable controller. In such a case, the driver also includes an input device, such as a keypad and a display.

In administering CPAP to a patient, the novel method includes the steps of providing a controllable flow of gas from a source, controlling the flow of gas so that the flow is periodically modified to inject a sigh cycle into the CPAP and providing the controlled flow of gas to said patient. In such a method, it is also preferred to attach a gas delivery device to the patient, wherein the gas delivery device has a breathing channel formed therein. In such a situation, the step of providing the controlled flow of gas to the patient includes providing the controlled flow of gas to the gas delivery device.

It is preferred in the method to monitor the airway pressure of the patient and periodically modifying the flow of gas in response to the monitoring of the airway pressure. In such a situation, it is especially preferred to determine when a drop is pressure is occurring and to periodically modify the gas flow to inject a sigh cycle into the CPAP in response to such pressure drop determination.

The method of the present invention can also be used to treat respiratory dysfunction in a patient and to stimulate the release of surfactant in immature lungs.

### BRIEF DESCRIPTION OF THE DRAWINGS

The present invention will be better understood, and its numerous objects and advantages will become apparent to those skilled in the art by reference to the following detailed description of the invention when taken in conjunction with the following drawings, in which:
Fig. 1 is a diagrammatic perspective view a generator presently available for use in administering NCPAP;
Fig. 2 is a graph of airway pressure versus time during the administration of NCPAP using the device depicted in Fig. 1;
Fig. 3 is block diagram of a system for injecting sighs during the administration of CPAP constructed in accordance with the present invention; and
Fig. 4 is a graph of airway pressure versus time during the administration of NCPAP using the device depicted in Fig. 3.

### DETAILED DESCRIPTION OF THE PREFERRED EMBODIMENTS

Referring to the drawings wherein like numerals indicate like elements throughout, there is shown in Fig. 1 a gas delivery device 10 presently used in administering NCPAP. Delivery device 10 is more commonly called a generator and will be so referenced herein. Generator 10 includes breathing channels 12a and 12b and gas inlet channels 14a and 14b. Nasal prongs 16a and 16b are attached to device 10 at one end of breathing channels 12a and 12b, respectively. Nasal prongs 16a and 16b are designed to fit within the nostrils of a newborn. It is noted that gas applied to inlet channels 14a and 14b intersects channels 12a and 12b in the manner and with the benefits disclosed in U.S. Patent No. 5,193,532. The provision of gas through inlet channels 14a and 14b enables device 10 to administer NCPAP, i.e., to generate a continuous positive airway pressure condition. A further channel 20 is also formed in device 10. Channel or passage 20 is in fluid communication with the ends of breathing channels 12a and 12b in a location proximate nasal prongs 16a and 16b. Since channel 20 is in fluid communication with breathing channels 12a and 12b, sensing or monitoring the pressure in channel 20 permits one to also determine the airway pressure.

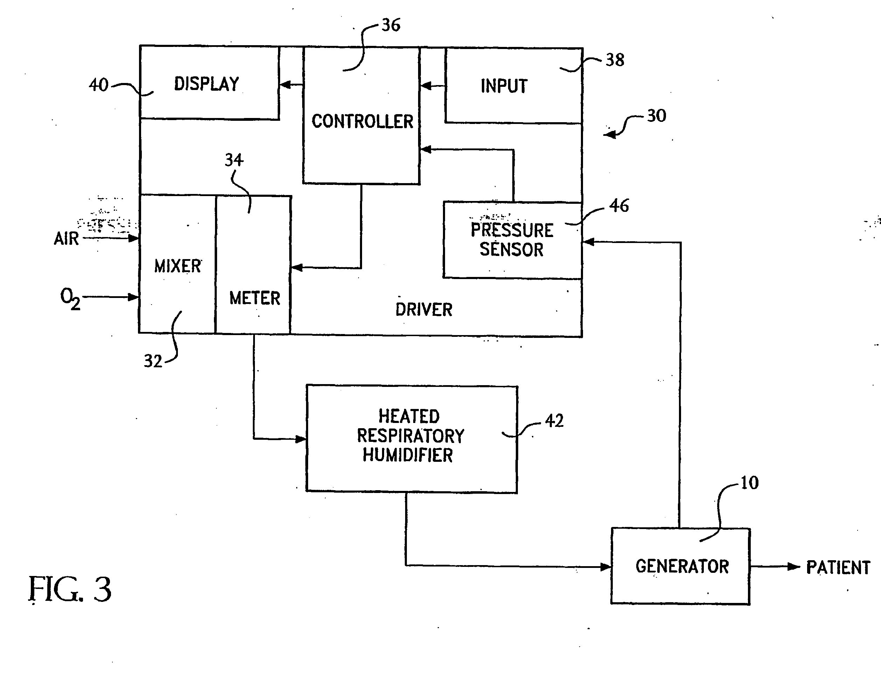

Referring now to Fig. 3, a driver 30 constructed in accordance with the present invention will be described. Driver 30 is adapted to be connected to a source of air and a source of oxygen. The air and oxygen are connected by any suitable structure to mixer 32. Mixer 32 acts to control the proportions of air and oxygen. Typically, such a mixer controls the percent oxygen. Mixer 32 is coupled to meter or valve mechanism 34. In one embodiment, valve mechanism 34 includes a single controllable valve. In such an embodiment, valve 34 is a controllable in response to a control signal so that more or less mixed gas is metered or permitted to flow. The control signal for controlling valve 34 is generated by controller 36. Controller 36 is connected to both an input device 38 and a display 40. In the preferred embodiment, controller 36 is programmable, with such programming being generated or made available via input device 38. Accordingly, input device 38 can be any form of apparatus for providing programming to controller 36, for example, a keyboard, a disk drive, electronic memory, etc.

Alternatively, valve 34 can include more than one valve connected so that opening both valves at the same time has an additive effect. For example, one valve would be operative for administering normal CPAP. Opening the second valve would produce a step increase in flow or pressure during the sigh or deep breath cycle. In yet another embodiment, each valve would be controlled to maintain CPAP pressure and different levels. When one valve is open, normal CPAP is administered. When that vale is closed and the other valve is opened, the sigh or deep breath cycle is administered.

Mixed gas, the flow of which is controlled by valve 34 is provided by any conventional structure, such as plastic tubing, to heated respiratory humidifier 42. The function of humidifier 42 is to control the humidity and temperature of the mixed gas being supplied to generator 10. No particular heated respiratory humidifier is required in order to practice the invention. If it is desired to control the temperature and humidity of the mixed gas being provided to generator 10, any known heated respiratory humidifier will be sufficient. Consequently, no further description of this device will be given. The output of mixed gas, processed by humidifier 42 is provided to generator 10. If generator 10 is of the type depicted in Fig. 1, the output of humidifier 42 is provided by any conventional structure, such as plastic tubing, to inlet 44 (Fig. 1).

A pressure sensor 46 is connected to sense the pressure in passage 20. In the preferred embodiment such a connection is made by establishing fluid communication between sensor 46 and inlet 48 on generator 10. Such fluid communication can be achieved by any conventional structure such as plastic tubing.

It is noted that the above described apparatus is utilized for the purpose of injecting sighs or deep breaths during the administration of CPAP, preferably NCPAP. Prior to the present invention, it was believed that the benefits of CPAP were most effectively achieved when the airway pressure was kept as constant as possible at a preset CPAP level chosen by the treating physician. In this regard, the equipment described in Fig. 3 could be used to produce the airway pressure pattern depicted in Fig. 2. To that end, controller 36 would monitor the signal generated by pressure sensor 46 and adjust the control signal to valve 34 to increase of decrease the flow of mixed gas there through, thereby increasing or decreasing airway pressure.

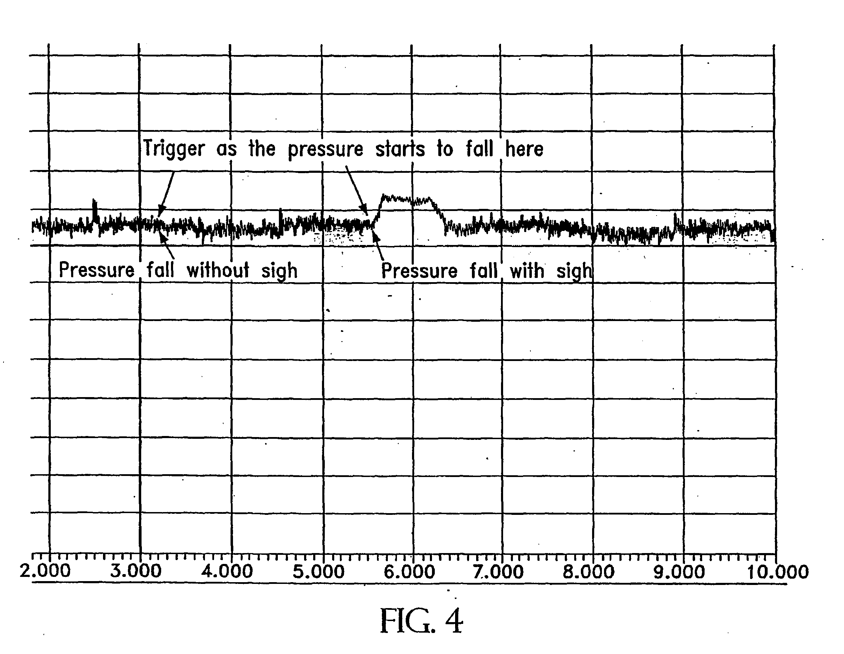

However, the present invention is designed to inject sigh cycles or deep breaths into the CPAP therapy. To this end, controller 36 monitors physiological conditions in order to determine the onset of inspiration, a breath. At that point, a sigh cycle or deep breath cycle is injected, after which the CPAP is returned to its previous level. In the embodiment depicted in Fig. 3, controller 36 monitors airway pressure to determine when such pressure is dropping, as shown in Fig. 4. When controller 36 determines that airway pressure is dropping, indicating that inspiration has begun, a sigh in injected.

It is noted that while it is preferable to inject a sigh at the moment inspiration begins, other points of injection are also within the scope of the invention. For example, any moment in time during the administration of CPAP can also be used. In such an embodiment, sighs will be injected into CPAP periodically, for example, so many sighs or deep breaths per minute. In such an embodiment, controller 36 will operate to determine when the appropriate time has passed and determine the appropriate time to modify the control signal to valve 34.

It is again noted, that it is not essential to the practice of the present invention to measure pressure in the breathing channel, i.e., airway pressure. It is only preferred that one determine the onset of inspiration. This condition could be determined in many ways, for example, using a flow sensor to measure either flow rate or flow direction. An electrical impedance measuring or sensing device can be connected to measure chest impedance changes during chest wall expansion and contraction. Such impedance changes are indicative of the onset of inspiration. Other alternatives to determine the onset of inspiration include the use of diaphragmatic EMG, inductance plethysmogaphy or any other volume or flow condition that would indicate inspiration.

Although, until now, NCPAP has been shown to provide significant benefits for newborns, there are remaining benefits that may be realized by the injection of intermittent sighs or deep breaths during NCPAP. Sighs are naturally occurring physiologic mechanisms that provide several benefits to newborns. Sighs are associated with stimulation of the release of surfactant, a biochemical substance that is responsible for stabilizing the alveoli of the lungs. This is important for the premature infant on a CPAP system, as they frequently have insufficient or immature surfactant systems.

Moreover, sighs will off load some of the work of breathing as the deep breath of the sigh will assist with the increased removal of carbon dioxide, potentially open some collapsed alveoli to improve oxygenation, as well as, stimulate the immature central respiratory system of the premature newborn to generate signals to the respiratory muscles to move the lungs for respiration. The injection of sighs will also act to stimulate the immature central respiratory system of the premature newborn, much the same as the current practice healthcare professionals to provide some external stimulus such as pinching.

### Example 1

A test of the invention was performed on a 1,003 gram newborn that was born eleven weeks prematurely and was three days old when placed on the system. The newborn had received NCPAP since birth, was given no surfactant and was down to an NCPAP pressure of 2. 5 cm of water with an inspired oxygen concentration of 21 % (equivalent to room air). The newborn's respiratory rate at the NCPAP pressure of 2.5 cm of water was 55 per minute and oxygen level by non-invasive saturation measurement was 98 %. The newborn was placed on a sigh rate of 10 per minute, i.e., 10 sighs were injected into the NCPAP per minute. After one hour the respiratory rate slowed to 47 per minute while the oxygen level remained normal at 99%. This slowing of the newborn's spontaneous respiratory rate indicates that the injection of sighs provided additional off loading of the ventilatory work, thereby decreasing the ventilatory work that the newborn had to do on its own. The sigh rate was then changed to 20 per minute and the baby left for thirty minutes. The respiratory rate decreased further to 43 per minute while the oxygen level remained at 99%. The slight increase in oxygen saturation from 98 to 99 percent following the institution of sigh breath injections may reflect some improvement in alveoli recruitment. It is noted that this application of the invention did not involve monitoring airway pressure to detect the onset of inspiration.

The present invention is useful in treating respiratory dysfunction in a patient and/or for stimulating the release of surfactant in immature lungs. To these ends the following method is utilized:
administering CPAP to the patient so that a continuous positive airway pressure is caused; monitoring the airway pressure of the patient; and modifying the CPAP to inject a sigh cycle in response to the monitoring of the airway pressure.

While the invention has been described and illustrated with reference to specific embodiments, those skilled in the art will recognize that modification and variations may be made without departing from the principles of the invention as described herein above and set forth in the following claims.

### CONCEPTS

As short summaries, this writing has disclosed at least the following concepts.
Concept 1. A driver for use in connection with a gas delivery device for the generation of a continuous positive airway pressure in a patient, wherein said gas delivery device has a breathing channel formed therein and is adapted for attachment to said patient, said driver being adapted for connection to a source of gas and said driver being adapted for connection with said gas delivery device, said driver comprising:
   a valve mechanism, for receiving gas from said source, wherein said valve, in response to a control signal, regulates the flow of said gas from said source to said gas delivery device; and
   a controller, connected to said valve, for generating said control signal so that said valve periodically modifies said flow of gas to said delivery device.
Concept 2. The driver of Concept 1, wherein said valve mechanism comprises a single controllable valve.
Concept 3. The driver of Concept 1, wherein said valve mechanism comprises more than one valve.
Concept 4. The driver of Concept 1, further comprising a sensor, connected to sense the pressure in said breathing channel, and connected to said controller, for generating a pressure signal representative of the pressure in said breathing channel, wherein said pressure signal is provided to said controller, wherein said controller periodically modifies said flow of gas in response to said pressure signal.
Concept 5. The driver of Concept 1, further comprising a sensor, connected to sense a preselected physiological condition, and connected to said controller, for generating a monitoring signal representative of a physiological condition in said patient, wherein said monitoring signal is provided to said controller, wherein said controller periodically modifies said flow of gas in response to said monitoring signal.
Concept 6. The driver of Concept 5, wherein said sensor in constructed to sense the onset of inspiration by said patient.
Concept 7. The driver of Concept 5, wherein said sensor in constructed to sense the flow of gas to said patient.
Concept 8. The driver of Concept 5, wherein said sensor in constructed to sense the electrical impedance in the chest of said patient.
Concept 9. The driver of Concept 5, wherein said sensor in constructed to sense the diaphramatic EMG.
Concept 10. The driver of Concept 5, wherein said sensor in constructed to sense inductance plethysmogaphy.
Concept 11. The driver of Concept 4, wherein said controller is constructed to monitor said pressure signal, to determine when a drop is pressure is occurring and to cause said valve mechanism to modify said flow of gas in response to such pressure drop determination.
Concept 12. The driver of Concept 4, wherein said controller is constructed to monitor said pressure signal, to determine when a drop is pressure is occurring and to cause said valve mechanism to administer a sigh cycle in response to such pressure drop determination.
Concept 13. The driver of Concept 1, wherein said controller comprises a programmable controller and wherein said driver further comprises an input device connected to said controller and a display connected to said controller.
Concept 14. The driver of Concept 1, further comprising a mixer, wherein said mixer is adapted to be connected to first and second sources of gas, for mixing gas from said first and second sources and for providing mixed gas to said valve.
Concept 15. A method for administering CPAP to a patient, said method comprising the steps of:
   providing a controllable flow of gas from a source;
   controlling said flow of gas so that said flow is periodically modified to inject a sigh cycle into said CPAP; and
   providing said controlled flow of gas to said patient.
Concept 16. The method of Concept 15, further comprising the step of attaching a gas delivery device to said patient, wherein said gas delivery device has a breathing channel formed therein, wherein said step of providing said controlled flow of gas to said patient, comprises providing said controlled flow of gas to said gas delivery device.
Concept 17. The method of Concept 15, further comprising the step of monitoring the airway pressure of said patient, wherein said step of controlling said flow of gas so that said flow is periodically modified to inject a sigh cycle into said CPAP, comprises periodically modifying said flow of gas in response to the monitoring of said airway pressure.
Concept 18. The method of Concept 17, wherein said step of monitoring the airway pressure of said patient, comprises the step of determining when a drop is pressure is occurring and wherein said step of controlling said flow of gas so that said flow is periodically modified to inject a sigh cycle into said CPAP is carried out in response to such pressure drop determination.
Concept 19. A method for treating respiratory dysfunction in a patient, said method comprising the steps of:
   administering CPAP to said patient such that a continuous positive airway pressure is caused;
   monitoring the airway pressure of said patient; and
   modifying said CPAP to inject a sigh cycle in response to the monitoring of said airway pressure.
Concept 20. A method for stimulating the release of surfactant in the immature lungs of a patient, said method comprising the steps of:
   administering CPAP to said patient such that a continuous positive airway pressure is caused;
   monitoring the airway pressure of said patient; and
   modifying said CPAP to inject a sigh cycle in response to the monitoring of said airway pressure.
Concept 21. Method for stimulating the respiratory center of a patient, said method
   comprising the steps of:
   administering CPAP to said patient such that a continuous positive airway pressure is caused;
   monitoring the airway pressure of said patient; and
   modifying said CPAP to inject a sigh cycle in response to the monitoring of said airway pressure.

## Claims

1. A system for treating respiratory dysfunction in a patient, said system comprising:
means for administering CPAP to said patient such that a continuous positive airway pressure is caused;
means for monitoring the airway pressure of said patient; and
means for modifying said CPAP to inject a sigh cycle in response to the monitoring of said airway pressure.

2. The system of Claim 1, further providing means for providing a controllable flow of gas from a source, wherein said means for modifying said CPAP to inject a sigh cycle in response to the monitoring of said airway pressure comprise means for controlling said flow of gas so that said flow is periodically modified to inject a sigh cycle into said CPAP in response to the means for monitoring said airway pressure.

3. The system of Claim 2, wherein said means for monitoring the airway pressure of said patient comprises:
means for determining when a drop is pressure is occurring and means for controlling said flow of gas so that said flow is periodically modified to inject a sigh cycle into said CPAP in response to such pressure drop determination.

4. The system of claim 1, comprising means for providing a controllable flow of gas from a source; and means for providing said controlled flow of gas to said patient.

5. The system of Claim 4, further comprising means for attaching a gas delivery device to said patient, wherein said gas delivery device has a breathing channel formed therein, wherein said means for providing said controlled flow of gas to said patient, comprises means for providing said controlled flow of gas to said gas delivery device.

6. The system of claim 1, wherein the system is capable of stimulating the release of surfactant in the immature lungs of a patient.

7. The system of claim 1, wherein the system is capable of stimulating the respiratory center of a patient.
